# EUROPÄISCHE PATENTANMELDUNG

(11) **EP 4 686 664 A1**
(43) Veröffentlichungstag der Anmeldung: **04.02.2026**
(21) Anmeldenummer: 25192792.7
(22) Anmeldetag: 30.07.2025
(51) Int. Cl.: B65D 5/66, A61F 15/00

(54) **VERBANDSTOFFVORRICHTUNG**

(30) Priorität: 30.07.2024 LU 507887
(71) Anmelder: W. Söhngen GmbH, 65232 Taunusstein (DE)
(72) Erfinder: Harms, Andreas, 26160 Bad Zwischenahn (DE)
(74) Vertreter: Hoffmann, Jürgen

(57) **Zusammenfassung**

Die Erfindung betrifft eine Verbandstoffvorrichtung mit einem Verbandstoffbehälter, der einen Stapel von eingesiegelten Pflasterabschnitten beinhaltet, wobei der Verbandstoffbehälter als Faltschachtel ausgebildet ist und im geschlossenen Zustand eine quaderförmige Form mit einer Länge l, einer Höhe h und einer Breite b, aufweist, wobei gilt: Höhe h < Breite b < Länge l. Die Verbandstoffvorrichtung zeichnet sich dadurch aus, dass eine Seite des Verbandstoffbehälters, die die Höhe h und die Länge l aufweist, als Klappdeckel ausgebildet ist oder Teil eines Klappdeckels ist, mittels dem ein Faltschachtelkörper verschließbar ist, und dass eine Verriegelungsvorrichtung vorhanden ist, die den Klappdeckel bei jedem Schließvorgang automatisch in der geschlossenen Stellung verriegelt.

## Beschreibung

Die Erfindung betrifft eine Verbandstoffvorrichtung mit einem Verbandstoffbehälter, der einen Stapel von eingesiegelten Pflasterabschnitten beinhaltet, wobei der Verbandstoffbehälter als Faltschachtel ausgebildet ist und im geschlossenen Zustand eine quaderförmige Form mit einer Länge I, einer Höhe h und einer Breite b, aufweist, wobei gilt: Höhe h < Breite b < Länge l.

Pflasterabschnitte sind typischerweise in einfachen, rechteckigen Kartons verpackt. Oftmals weisen die Kartons keine Möglichkeit auf, nach der Entnahme eines Pflasterabschnittes effizient und zuverlässig wieder verschlossen zu werden. Dies kann dazu führen kann, dass die Verpackung offen bleibt oder dass sich die Verpackung ungewollt öffnet und Pflasterabschnitte herausfallen, wodurch die Pflasterabschnitte verunreinigt werden oder verloren gehen.

Es gibt auch Verbandstoffvorrichtungen, bei denen die Pflasterabschnitte nur umständlich aus dem Verbandstoffbehälter entnehmbar sind. Oftmals ist die Entnahme derart umständlich, dass während der Entnahme eines der Pflasterabschnitte ungewollt weitere Pflasterabschnitte aus dem Karton gezogen werden, die anschließend wieder umständlich zurück in den Karton gesteckt werden müssen.

Ein besonderer Nachteil herkömmlicher Verbandstoffvorrichtungen ist insoweit ihre oftmals umständliche Handhabung in Notfallsituationen, in denen ein schnelles und sicheres Öffnen, eine schnelle und sichere Entnahme und ein einfaches und sicheres Wiederverschließen erforderlich ist.

Aus DE 89 12 948 U1 ist eine quaderförmige Faltschachtel bekannt, deren Inhalt staubgeschützt und diebstahlsicher dargeboten werden kann. Bei dieser Faltschachtel ist wesentlich, dass ein mit einer Aufreißlasche verbundener Originalitäts- und diebstahlssicherer Verschluss auf einer vorderen und/oder unteren Seitenwandfläche der Faltschachtel ausgebildet ist. Im Originalzustand ist eine Aufbrechlasche vermittels einer Schwächungs- oder Sollbruchlinie in der Seitenwand der Faltschachtel gehalten. Durch Eindrücken der Aufbrechlasche wird diese Schwächungsund Sollbruchlinie zerstört, so dass ein Öffnen der Faltschachtel möglich ist.

Aus WO 2015 017756 A1 offenbart einen Kartonbehälter zum Aufbewahren eines Produkts. Der Kartonbehälter umfasst mehrere Wandungen, die sich zumindest teilweise um das Innere erstrecken. Die mehreren Wandungen umfassen eine Vorderwand, eine Rückwand, eine erste Seitenwand und eine zweite Seitenwand. Der Kartonbehälter umfasst ferner ein Scharnier in mindestens einer der mehreren Wandungen und einen wiederverschließbaren Deckel, der schwenkbar an dem Kartonbehälter angebracht ist. Der wiederverschließbare Deckel umfasst eine erste Zwischenwand, die faltbar mit mindestens einer der mehreren Wände verbunden ist, und eine zweite Zwischenwand, die faltbar mit der ersten Zwischenwand verbunden ist. Die erste Zwischenwand ist so positioniert, dass sie mit der zweiten Zwischenwand in direktem Kontakt steht. Der wiederverschließbare Deckel ist an dem Scharnier zwischen einer geschlossenen Position und einer offenen Position schwenkbar angeordnet.

Es ist daher die Aufgabe der vorliegenden Erfindung, eine Verbandstoffvorrichtung anzugeben, die eine einfache Handhabung ermöglicht.

Die Aufgabe wird durch eine Verbandstoffvorrichtung der eingangs genannten Art gelöst, die dadurch gekennzeichnet ist, dass eine Seite des Verbandstoffbehälters, die die Höhe h und die Länge l aufweist, als Klappdeckel ausgebildet ist oder Teil eines Klappdeckels ist, mittels dem ein Faltschachtelkörper verschließbar ist, und dass eine Verriegelungsvorrichtung vorhanden ist, die den Klappdeckel bei jedem Schließvorgang automatisch in der geschlossenen Stellung verriegelt.

Die erfindungsgemäße Verbandstoffvorrichtung hat den ganz besonderen Vorteil, dass der Verbandstoffbehälter einfach, insbesondere auch mit einer einzigen Hand, durch Aufklappen des Klappdeckels geöffnet werden kann, eingesiegelte Pflasterabschnitte einzeln einfach entnommen werden können einfach und zuverlässig beim Schließvorgang wieder verschlossen werden kann, wobei keine zusätzlichen Handlungen zum Verriegeln des Klappdeckels in der geschlossenen Stellung erforderlich ist. Hierbei kann vorteilhaft vorgesehen sein, dass der Schließvorgang ausschließlich ein einfaches Zuklappen des Klappdeckels beinhaltet.

Die automatische Verriegelungsvorrichtung sorgt dafür, dass der Verbandstoffbehälter solange sicher verschlossen bleibt, bis wieder ein Pflasterabschnitt benötigt wird, was den Schutz der in dem Verbandstoffbehälter aufbewahrten Pflasterabschnitte gewährleistet.

Dadurch, dass eine Seite des Verbandstoffbehälters, die die Höhe h und die Länge l aufweist, als Klappdeckel ausgebildet ist oder Teil eines Klappdeckels ist, mittels dem ein Faltschachtelkörper verschließbar ist, wird vorteilhaft ein, vorteilhafter Weise auch einhändiges, einfaches Greifen und Entnehmen einzelner Pflasterabschnitte ermöglicht, wodurch vorteilhaft vermieden ist, dass während der Entnahme eines der Pflasterabschnitte ungewollt weitere Pflasterabschnitte aus dem Karton herausgezogen werden, die anschließend wieder umständlich zurück in den Verbandstoffbehälter gesteckt werden müssen. Außerdem erlaubt der erfindungsgemäße Verbandstoffbehälter eine einfache Entnahme eines Pflasterabschnitts, ohne dass alle oder mehrere Pflasterabschnitte erst herausgehoben oder herausgekippt werden müssen. Es hat sich beispielsweise gezeigt, dass diese Vorteile weitgehend nicht oder nur in sehr vermindertem Maße eintreten, wenn eine Seite des Verbandstoffbehälters, die die Höhe h und die Breite b aufweist, als Klappdeckel ausgebildet ist oder Teil eines Klappdeckels ist, mittels dem ein Faltschachtelkörper verschließbar ist. Auch wenn eine Seite, die die Länge l und die Breite b aufweist, als Klappdeckel ausgebildet ist oder Teil eines Klappdeckels ist, also wenn der Verbandstoffbehälters truhenförmig ausgebildet ist, ist ein problemloses, insbesondere einhändiges, Entnehmen von Pflasterabschnitten gegenüber einem Entnehmen aus dem erfindungsgemäßen Verbandstoffbehälter deutlich erschwert.

Der Klappdeckel ist vorzugsweise mittels eines Deckelscharniers mit dem ein Faltschachtelkörper verbunden. Das Deckelscharnier kann durch Knicken des Faltschachtelmaterials als eine Art Filmscharnier hergestellt sein. Das Deckelscharnier ist vorzugsweise derart ausgebildet, dass der Klappdeckel um eine parallel zur Länge l verlaufende Schwenkachse relativ zu dem Faltschachtelkörper geschwenkt werden kann.

Bei einer ganz besonders vorteilhaften Ausführung ist die Faltschachtel samt der Verriegelungsvorrichtung aus einem einzigen Stück Karton oder Pappe hergestellt ist. Dies reduziert die Produktionskosten und vereinfacht den Herstellungsprozess, da wenig Materialien und wenige Verarbeitungsschritte zur Herstellung der Faltschachtel erforderlich sind. Zudem fördert diese Herstellungsweise die Recyclingfähigkeit des Verbandstoffbehälters, was umweltfreundlich ist.

Besonders vorteilhaft ist eine Ausführung, bei der die Verriegelungsvorrichtung eine, insbesondere schwenkbar angeordnete, Verriegelungszunge aufweist. Eine solche Ausführung der Verriegelungsvorrichtung ist einfach herstellbar. Außerdem erlaubt eine solche Verriegelungsvorrichtung dennoch einfache und zuverlässige Verriegelung, die gleichzeitig benutzerfreundlich ist und die Integrität der Verbandstoffbehälters auch nach häufigem Öffnen und Schließen bewahrt.

Insbesondere kann vorteilhaft vorgesehen sein, dass die Verriegelungszunge an dem Faltschachtelkörper ausgebildet ist. Dies trägt zur Stabilität und Haltbarkeit der Verriegelungsvorrichtung bei, da sie mit dem Faltschachtelkörper der Faltschachtel verbunden ist und insoweit von diesem gestützt wird und ihrerseits zur Stabilität des Faltschachtelkörpers beiträgt.

Besonders vorteilhaft ist eine Ausführung, bei der die Verriegelungszunge elastisch biegbar ausgebildet ist. Dies ermöglicht eine wiederholte Nutzung der Verriegelungsvorrichtung ohne Materialermüdung und gewährleistet eine dauerhafte Funktionalität.

Die Verbandstoffvorrichtung kann vorteilhaft so gestaltet sein, dass sich die Längserstreckungsrichtung der Verriegelungszunge in Richtung der Länge l erstreckt. Dies sorgt für eine gleichmäßige Verteilung der Verriegelungskraft und gewährleistet, dass der Klappdeckel weitgehend entlang der gesamten Länge l sicher und zuverlässig in der geschlossenen Stellung verriegelt wird.

Besonders vorteilhaft ist eine Ausführung, bei der die Längserstreckungsrichtung der Verriegelungszunge parallel zu dem Deckelscharnier ausgerichtet ist, welches zwischen dem Faltschachtelkörper und dem Klappdeckel ausgebildet ist. Dies ist besonders vorteilhaft für die strukturelle Integrität und Funktionalität der Verriegelungsvorrichtung.

Bei einer ganz besonders vorteilhaften Ausführung weist der Klappdeckel einen Verriegelungsabsatz auf, an dem die Verriegelungszunge, insbesondere eine freie Stirnseite der Verriegelungszunge, bei jedem Schließvorgang einrastet. Dies gewährleistet eine sichere und feste Verriegelung, die leicht zu bedienen ist. Die Verriegelungsvorrichtung verriegelt so den Klappdeckel bei jedem Schließvorgang, der ein Schwenken des Klappdeckels in die geschlossene Stellung beinhaltet, automatisch in der geschlossenen Stellung, indem die Verriegelungszunge bei jedem Schließvorgang automatisch hinter den Verriegelungsabsatz greift und dort einrastet. Zum Öffnen des Klappdeckels muss der Benutzer den Klappdeckel zurück in die geöffnete Stellung schwenken und dabei anfänglich die Haltekraft der Verriegelungszunge überwinden. Beim Öffnen des Klappdeckels biegt kann vorteilhaft vorgesehen sein, dass sich die Verriegelungszunge etwas biegt, so dass sie außer Eingriff von dem Verriegelungsabsatz gelangt und der Klappdeckel freigegeben wird.

Der Klappdeckel kann vorteilhaft einen Kragen aufweisen. Ein solcher Kragen kann dazu beitragen, den Inhalt des Behälters zusätzlich zu schützen und die Gesamtstabilität der Verpackung zu erhöhen. Außerdem bildet der Kragen in der geöffneten Stellung eine Barriere für die Pflasterabschnitte für den Fall, dass der Verbandstoffbehälter von dem Benutzer in der geöffneten Stellung versehentlich so schräg gehalten wird, dass einzelne Pflasterabschnitte der Gewichtskraft folgend aus dem Faltschachtelkörper rutschen.

Besonders vorteilhaft ist eine Ausführung, bei der der Kragen im geschlossenen Zustand an den Außenflächen des Faltschachtelkörpers anliegt. Dies sorgt für eine zusätzliche Barriere gegen das Eindringen von Schmutz und erhöht die Stabilität der Faltschachtel im geschlossenen Zustand.

Insbesondere kann vorteilhaft vorgesehen sein, dass der Verriegelungsabsatz an der Innenseite des Kragens ausgebildet ist. Diese Anordnung schützt die Verriegelungsvorrichtung insbesondere im geschlossenen Zustand vor äußeren Einflüssen, insbesondere vor einer Beschädigung und/oder einem ungewollten Öffnen, und gewährleistet eine zuverlässige Funktion über einen langen Zeitraum.

Die Verbandstoffvorrichtung kann vorteilhaft so gestaltet sein, dass der Kragen eine Frontlasche und zwei Seitenlaschen aufweist, wobei die Seitenlaschen jeweils einen Verbindungsabschnitt aufweisen, der von innen gegen die Frontlasche geklebt ist. Dies erhöht die Stabilität der Verpackung und insbesondere des Klappdeckels und des Kragens.

Besonders vorteilhaft ist eine Ausführung, bei der eine Stirnseite eines der Verbindungsabschnitte den Verriegelungsabsatz bildet oder bei der jeweils eine Stirnseite jedes der Verbindungsabschnitte Teil eines Verriegelungsabsatzes ist, hinter dem die Verriegelungszunge bei jedem Schließvorgang automatisch greift und dort einrastet. Dies sorgt für eine robuste und zuverlässige Verriegelungsvorrichtung, die einfach zu produzieren ist.

Alternativ kann vorteilhaft vorgesehen sein, dass der Verriegelungsabsatz, hinter dem die Verriegelungszunge bei jedem Schließvorgang automatisch greift und dort einrastet, durch die freie Stirnseite einer nach innen umgeschlagenen Umschlaglasche der Frontlasche gebildet ist. Auch eine solche Ausführung ist besonders robust und zuverlässig.

Vorzugsweise weisen die Pflasterabschnitte jeweils eine im Wesentlichen flache, vorzugsweise rechteckige, Form auf.

Vorzugsweise sind die Pflasterabschnitte derart in dem Verbandstoffbehälter gestapelt, dass sie parallel zu den Seiten des Verbandstoffbehälters angeordnet sind, die Breite b und die Länge l aufweisen.

Die Verbandstoffvorrichtung kann vorteilhaft Pflasterabschnitte umfassen, die mittels einer rechteckigen flachen, insbesondere biegsamen Hülle eingesiegelt sind. Diese Verpackungsform bietet eine flexible und dennoch schützende Hülle für die Pflaster, die leicht zu handhaben und zu öffnen ist. Insbesondere ist eine solche Verpackungsform besonders gut geeignet, um die Pflasterabschnitte für eine einfache Entnahme ordentlich gestapelt in dem Verbandstoffbehälter aufzubewahren

Vorzugsweise sind die Hüllen derart in dem Verbandstoffbehälter gestapelt, dass sie parallel zu den Seiten des Verbandstoffbehälters angeordnet sind, die Breite b und die Länge l aufweisen.

Besonders vorteilhaft ist eine Ausführung, bei der die Hülle eine Hüllenlänge hl und eine Hüllenbreite hb aufweist, wobei gilt 0,9 I < hl < 0,99 l und 0,9 b < hb < 0,99 b. Diese Dimensionierung sorgt dafür, dass eine effiziente Raumnutzung gewährleistet ist und die Pflasterabschnitte bei dem Transport der Verbandstoffvorrichtung nicht durcheinander geraten. Insbesondere kann vorteilhaft vorgesehen sein, dass die Länge l im Bereich von 5 cm bis 20 cm, insbesondere im Bereich von 6 cm bis 12 cm liegt. Diese Dimensionierung ermöglicht eine handliche und portable Verpackung, die dennoch ausreichend Pflaster aufnehmen kann.

Der Verbandstoffbehälter kann vorteilhaft eine Breite b im Bereich von 3 cm bis 15 cm, insbesondere im Bereich von 4 cm bis 8 cm aufweisen. Dies sorgt für eine kompakte und leicht zu verstauende Verbandstoffvorrichtung, die besonders benutzerfreundlich ist.

Ganz besonders vorteilhaft ist eine Ausführung, bei der der Verbandstoffbehälter dazu ausgebildet ist, mit einer einzigen Hand gehalten, geöffnet und wieder geschlossen zu werden. Dies erhöht die Benutzerfreundlichkeit erheblich, insbesondere in Notfallsituationen, in denen eine schnelle und einfache Handhabung entscheidend ist. Insbesondere hierfür kann der Verbandstoffbehälter vorteilhaft die oben genannten Abmessungen aufweisen.

Es ist nach einem besonderen Erfindungsgedanken insbesondere auch möglich, dass der Verbandstoffbehälter anstelle von dem Stapel von eingesiegelten Pflasterabschnitten andere Medizinprodukte, wie sterile Kompressen, Verbandtücher, Desinfektionstücher und/oder Einweg-Injektionsnadeln, beinhaltet.

Besonders vorteilhaft ist ein Verbandstoffbehälter für eine erfindungsgemäße Verbandstoffvorrichtung.

Der Verbandstoffbehälter ist nicht nur ideal für die Aufbewahrung von Pflasterabschnitten, sondern kann auch vorteilhaft zur Aufbewahrung anderer Produkte verwendet werden. Beispiele für medizinische Produkte, die in diesem Behälter aufbewahrt werden können, sind, wie bereits erwähnt, sterile Kompressen, Verbandtücher, Desinfektionstücher und/oder Einweg-Injektionsnadeln. Diese Produkte profitieren von der sicheren und handlichen Aufbewahrung in dem Verbandstoffbehälter. Nichtmedizinische Produkte, die ebenfalls in dem Verbandstoffbehälter aufbewahrt werden können, können beispielsweise Büroklammern, Stecknadeln, Batterien, Schmuckstücke, kleine elektronische Bauteile oder Kaugummis umfassen. Der robuste Verschlussmechanismus und die widerstandsfähige Bauweise des Behälters sorgen dafür, dass diese Gegenstände sicher und geordnet verstaubar sind und dennoch einfach und sicher, insbesondere einzeln, entnommen werden können.

Durch seine vielseitige Einsetzbarkeit bietet der erfindungsgemäße Verbandstoffbehälter eine praktische und flexible Lösung für verschiedene Aufbewahrungsbedürfnisse, sowohl im medizinischen als auch im nichtmedizinischen Bereich.

In der Zeichnung ist der Erfindungsgegenstand beispielhaft und schematisch dargestellt und wird anhand der Figuren nachfolgend beschrieben, wobei gleiche oder gleich wirkende Elemente auch in unterschiedlichen Ausführungsbeispielen zumeist mit denselben Bezugszeichen versehen sind. Dabei zeigen:
- Fig. 1: ein erstes Ausführungsbeispiel einer erfindungsgemäßen Verbandstoffvorrichtung in einer perspektivischen Ansicht bei geschlossenem Verbandstoffbehälter,
- Fig.2: das erste Ausführungsbeispiel einer erfindungsgemäßen Verbandstoffvorrichtung in einer perspektivischen Ansicht er bei geöffnetem Verbandstoffbehälter,
- Fig.3: das erste Ausführungsbeispiel einer erfindungsgemäßen Verbandstoffvorrichtung in einer Querschnittsdarstellung bei geschlossenem Verbandstoffbehälter,
- Fig.4: das erste Ausführungsbeispiel einer erfindungsgemäßen Verbandstoffvorrichtung in einer Querschnittsdarstellung bei geöffnetem Verbandstoffbehälter, und
- Fig.5: ein erstes Ausführungsbeispiel einer erfindungsgemäßen Verbandstoffvorrichtung in einer perspektivischen Ansicht bei geöffnetem Verbandstoffbehälter.

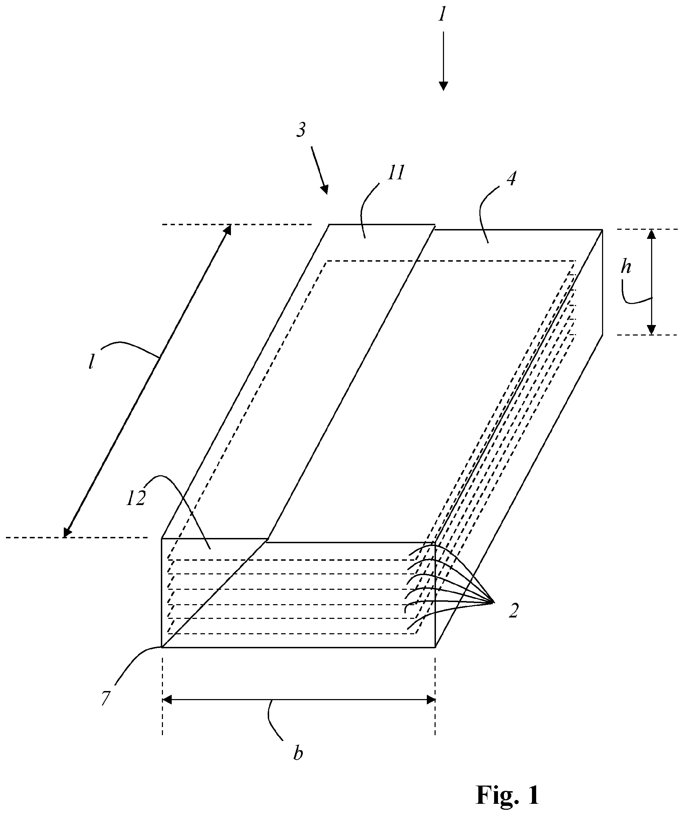

Die Figur 1 zeigt ein erstes Ausführungsbeispiel einer erfindungsgemäßen Verbandstoffvorrichtung.

Die Verbandstoffvorrichtung weist einen Verbandstoffbehälter 1 auf, der einen Stapel von eingesiegelten Pflasterabschnitten 2 beinhaltet. Der Verbandstoffbehälter 1 ist als Faltschachtel ausgebildet ist und weist im geschlossenen Zustand eine quaderförmige Form mit einer Länge l, einer Höhe h und einer Breite b, auf, wobei gilt: Höhe h < Breite b < Länge l.

Eine Seite des Verbandstoffbehälter, die die Höhe h und die Länge l aufweist, ist Teil eines Klappdeckels 3, mittels dem ein Faltschachtelkörper 4 verschließbar ist. Der Klappdeckels 3 ist mittels eines Deckelscharniers 7 mit dem ein Faltschachtelkörper 4 verbunden. Das Deckelscharnier 7 kann durch Knicken des Faltschachtelmaterials als eine Art Filmscharnier hergestellt sein.

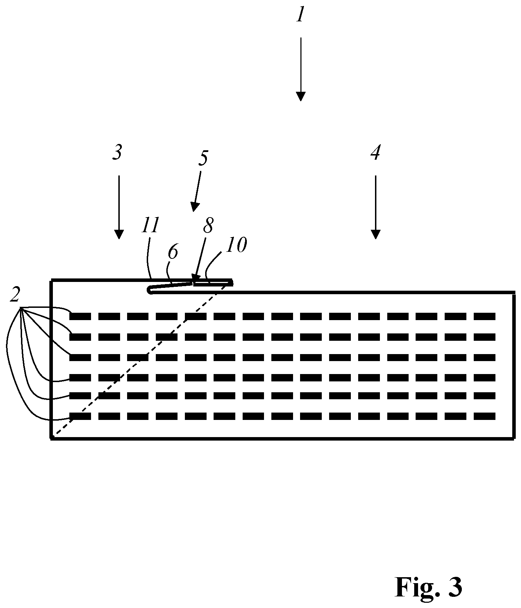

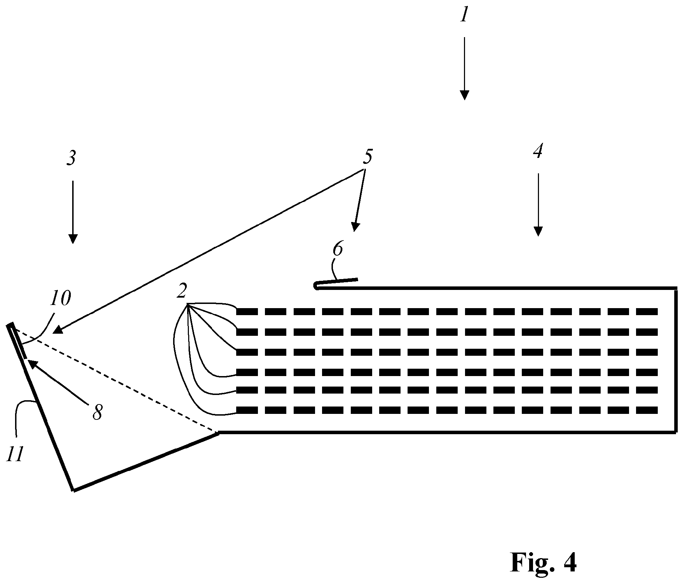

Es ist eine in der Figur 1 nicht sichtbare Verriegelungsvorrichtung 5 vorhanden, die den Klappdeckel 3 bei jedem Schließvorgang automatisch in der geschlossenen Stellung verriegelt. In der Figur 1 ist die Verbandstoffvorrichtung bei geschlossenem Verbandstoffbehälter 1 dargestellt. Die Funktionsweise der Verriegelungsvorrichtung 5 ist in den Figuren 3 und 4 illustriert und weiter unten erläutert.

Der Klappdeckel 3 weist einen Kragen 9 auf. Der Kragen 9 liegt im geschlossenen Zustand an den Außenflächen des Faltschachtelkörpers 4 an.

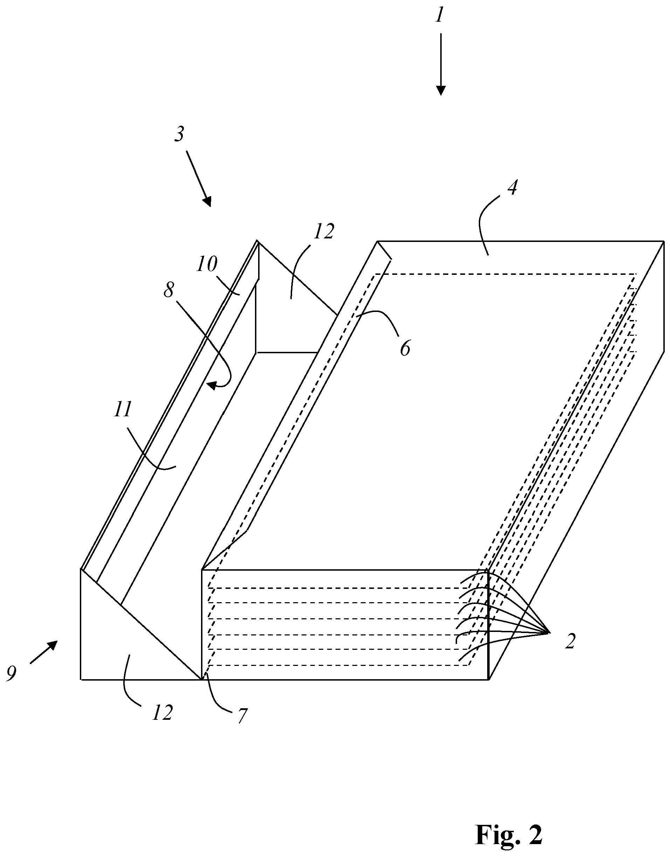

Die Figur 2 zeigt das erste Ausführungsbeispiel einer erfindungsgemäßen Verbandstoffvorrichtung in einer perspektivischen Ansicht bei geöffnetem Verbandstoffbehälter 1. Als Teil der Verriegelungsvorrichtung 5 weist die Verbandstoffvorrichtung eine schwenkbar angeordnete Verriegelungszunge 6 auf, die sich in Richtung der Länge l erstreckt und die parallel zu dem Deckelscharnier 7 ausgerichtet ist.

Der Klappdeckel 3 weist einen Verriegelungsabsatz 8 auf, an dem die freie Stirnseite der Verriegelungszunge 6 bei jedem Schließvorgang automatisch einrastet.

Der Verriegelungsabsatz 8 ist an der Innenseite des Kragens 9 ausgebildet. Konkret ist Verriegelungsabsatz 8 durch die freie Stirnseite einer nach innen umgeschlagenen Umschlaglasche 10 einer Frontlasche 11 des Kragens 9 gebildet. Der Kragen 9 weist außer der Frontlasche 11 zwei Seitenlaschen 12 auf.

Die Figuren 3 und 4 zweigen das erste Ausführungsbeispiel einer erfindungsgemäßen Verbandstoffvorrichtung in einer Querschnittsdarstellung bei geschlossenem Verbandstoffbehälter (Figur 3) und bei geöffnetem Verbandstoffbehälter (Figur 4) und illustrieren die Funktionsweise der Verriegelungsvorrichtung 5.

Die Verriegelungsvorrichtung 5 verriegelt den Klappdeckel 3 bei jedem Schließvorgang, der ein Schwenken des Klappdeckels 3 in die geschlossene Stellung beinhaltet, automatisch in der geschlossenen Stellung, indem die Verriegelungszunge 6 bei jedem Schließvorgang automatisch hinter den Verriegelungsabsatz 8 greift und dort einrastet, was in Figur 3 dargestellt ist.

Zum Öffnen des Klappdeckels 3 muss der Benutzer den Klappdeckel zurück in die geöffnete Stellung schwenken und dabei anfänglich die Haltekraft der Verriegelungszunge 6 überwinden. Beim Öffnen des Klappdeckels 3 biegt sich die Verriegelungszunge 6 etwas, so dass sie außer Eingriff von dem Verriegelungsabsatz 8 gelangt und der Klappdeckel 3 freigegeben wird, was in Figur 4 dargestellt ist.

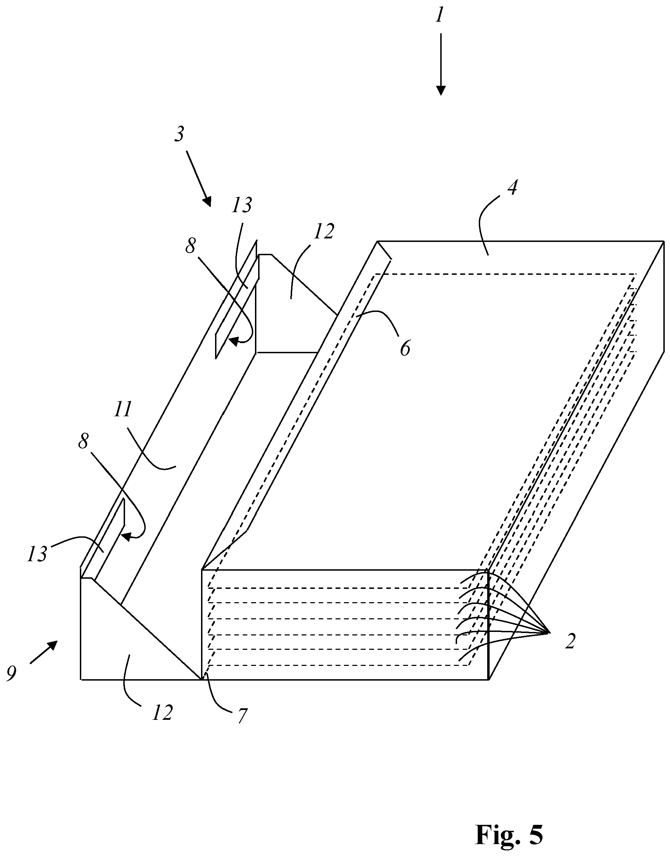

Die Figur 5 zeigt ein zweites Ausführungsbeispiel einer erfindungsgemäßen Verbandstoffvorrichtung in einer perspektivischen Ansicht bei geöffnetem Verbandstoffbehälter 1. Im Unterschied zu dem ersten Ausführungsbeispiel weisen die Seitenlaschen 12 des Kragens 9 jeweils einen Verbindungsabschnitt 13 auf, der von innen gegen die Frontlasche 11 geklebt ist. Bei dieser Ausführung bildet jeweils eine Stirnseite jedes der Verbindungsabschnitte 13 einen Verriegelungsabsatz 8.

Die Verriegelungsvorrichtung 5 verriegelt den Klappdeckel 3 auch bei dieser Ausführung bei jedem Schließvorgang automatisch in der geschlossenen Stellung, indem die Verriegelungszunge 6 bei jedem Schließvorgang automatisch hinter die Verriegelungsabsätze 8 greift und dort einrastet. Beim Öffnen des Klappdeckels 3 biegt sich die Verriegelungszunge 6 etwas, so dass sie außer Eingriff von den Verriegelungsabsätzen 8 gelangt und der Klappdeckel 3 freigegeben wird.

### Bezugszeichenliste:

- 1: Verbandstoffbehälter
- 2: Pflasterabschnitt
- 3: Klappdeckel
- 4: Faltschachtelkörper
- 5: Verriegelungsvorrichtung
- 6: Verriegelungszunge
- 7: Deckelscharnier
- 8: Verriegelungsabsatz
- 9: Kragen
- 10: Umschlaglasche
- 11: Frontlasche
- 12: Seitenlaschen
- 13: Verbindungsabschnitt

- l: Länge
- b: Breite
- h: Höhe

## Patentansprüche

1. Verbandstoffvorrichtung mit einem Verbandstoffbehälter (1), der einen Stapel von eingesiegelten Pflasterabschnitten (2) beinhaltet, wobei der Verbandstoffbehälter (1) als Faltschachtel ausgebildet ist und im geschlossenen Zustand eine quaderförmige Form mit einer Länge l, einer Höhe h und einer Breite b, aufweist, wobei gilt: Höhe h < Breite b < Länge l, **dadurch gekennzeichnet, dass** eine Seite des Verbandstoffbehälters (1), die die Höhe h und die Länge l aufweist, als Klappdeckel (3) ausgebildet ist oder Teil eines Klappdeckels (3) ist, mittels dem ein Faltschachtelkörper (4) verschließbar ist, und dass eine Verriegelungsvorrichtung (5) vorhanden ist, die den Klappdeckel (3) bei jedem Schließvorgang automatisch in der geschlossenen Stellung verriegelt.

2. Verbandstoffvorrichtung nach Anspruch 1, **dadurch gekennzeichnet, dass** die Faltschachtel samt der Verriegelungsvorrichtung (5) aus einem einzigen Stück Karton oder Pappe hergestellt ist.

3. Verbandstoffvorrichtung nach Anspruch 1 oder 2, **dadurch gekennzeichnet, dass** der Klappdeckel (3) mittels eines Deckelscharniers (7) mit dem ein Faltschachtelkörper (4) verbunden ist.

4. Verbandstoffvorrichtung nach Anspruch 3, **dadurch gekennzeichnet, dass**
a. das Deckelscharnier (7) durch Knicken eines Faltschachtelmaterials als eine Art Filmscharnier hergestellt ist, und/oder dass
b. das Deckelscharnier (7) derart ausgebildet ist, dass der Klappdeckel (3) um eine parallel zur Länge l verlaufende Schwenkachse relativ zu dem Faltschachtelkörper (4) schwenkbar ist.

5. Verbandstoffvorrichtung nach einem der Ansprüche 1 bis 4, **dadurch gekennzeichnet, dass** die Verriegelungsvorrichtung (5) eine, insbesondere schwenkbar angeordnete, Verriegelungszunge (6) aufweist.

6. Verbandstoffvorrichtung nach Anspruch 5, **dadurch gekennzeichnet, dass** die Verbandstoffvorrichtung wenigstens eines der folgenden Merkmale a bis e aufweist:
a. die Verriegelungszunge (6) ist an dem Faltschachtelkörper ausgebildet,
b. die Verriegelungszunge (6) ist elastisch biegbar ausgebildet,
c. die Längserstreckungsrichtung der Verriegelungszunge (6) erstreckt sich in Richtung der Länge l,
d. die Längserstreckungsrichtung der Verriegelungszunge (6) ist parallel zu dem Deckelscharnier (7) ausgerichtet,
e. der Klappdeckel (3) weist einen Verriegelungsabsatz (8) auf, an dem die Verriegelungszunge (6), insbesondere eine freie Stirnseite der Verriegelungszunge (6), bei jedem Schließvorgang einrastet.

7. Verbandstoffvorrichtung nach einem der Ansprüche 1 bis 6, **dadurch gekennzeichnet, dass** der Klappdeckel (3) einen Kragen (9) aufweist.

8. Verbandstoffvorrichtung nach Anspruch 7, **dadurch gekennzeichnet, dass** die Verbandstoffvorrichtung wenigstens eines der folgenden Merkmale a bis e aufweist:
a. der Kragen (9) liegt im geschlossenen Zustand an Außenflächen des Faltschachtelkörpers (4) an,
b. der Verriegelungsabsatz (8) ist an der Innenseite des Kragens (9) ausgebildet,
c. der Kragen (9) weist eine Frontlasche (11) und zwei Seitenlaschen (12) auf, wobei die Seitenlaschen (12) jeweils einen Verbindungsabschnitt (13) aufweisen, der von innen gegen die Frontlasche (11) geklebt ist,
d. eine Stirnseite eines der Verbindungsabschnitte (13) bildet den Verriegelungsabsatz (8) oder jeweils eine Stirnseite jedes der Verbindungsabschnitte (13) bildet einen Verriegelungsabsatz (8),
e. der Verriegelungsabsatz (8) ist durch eine Stirnseite eines freien Endes einer nach innen umgeschlagenen Umschlaglasche (10) der Frontlasche (11) gebildet.

9. Verbandstoffvorrichtung nach einem der Ansprüche 1 bis 8, **dadurch gekennzeichnet, dass** die Pflasterabschnitte (2) mittels einer rechteckigen flachen, insbesondere biegsamen, Hülle eingesiegelt sind.

10. Verbandstoffvorrichtung nach Anspruch 9, **dadurch gekennzeichnet, dass** die Hülle eine Hüllenlänge hl und eine Hüllenbreite hb und aufweist, wobei gilt 0,9 l < hl < 0,99 l und 0,9 b < hb < 0,99 b.

11. Verbandstoffvorrichtung nach einem der Ansprüche 1 bis 10, **dadurch gekennzeichnet, dass** die Länge l im Bereich von 5 cm bis 20 cm, insbesondere im Bereich von 6 cm bis 12 cm, liegt.

12. Verbandstoffvorrichtung nach einem der Ansprüche 1 bis 11, **dadurch gekennzeichnet, dass** die Breite b im Bereich von 3 cm bis 15 cm, insbesondere im Bereich von 4 cm bis 8 cm, liegt.

13. Verbandstoffvorrichtung nach einem der Ansprüche 1 bis 12, **dadurch gekennzeichnet, dass** der Verbandstoffbehälter (1) dazu ausgebildet ist, mit einer einzigen Hand gehalten, geöffnet und wieder geschlossen zu werden.

14. Verbandstoffvorrichtung nach einem der Ansprüche 1 bis 13, **dadurch gekennzeichnet, dass** der Verbandstoffbehälter (1) anstelle von dem Stapel von eingesiegelten Pflasterabschnitten (2) andere Medizinprodukte, wie sterile Kompressen, Verbandtücher, Desinfektionstücher und/oder Einweg-Injektionsnadeln, beinhaltet.

15. Verbandstoffbehälter (1) für eine Verbandstoffvorrichtung nach einem der Ansprüche 1 bis 14.
